# EUROPEAN PATENT APPLICATION

(11) **EP 1 344 522 A1**
(43) Date of publication of application: **17.09.2003**
(21) Application number: 03013353.2
(22) Date of filing: 08.05.2001
(51) Int. Cl.: A61K 9/70, A61K 31/381

(54) **Transdermal therapeutic system for Parkinson's disease inducing high plasma levels of rotigotine**

(62) Divisional of application: 01111109.3
(71) Applicant: SCHWARZ PHARMA AG, 40789 Monheim (DE); LTS Lohmann Therapie-Systeme AG, 56626 Andernach (DE)
(72) Inventor: Lauterbach, Thomas, Dr., 40595 Düsseldorf (DE); Schacht, Dietrich Wilhelm, 50935 Köln (DE); Wolff, Hans-Michael, Dr., 40789 Monheim (DE); Müller, Walter, Dr., 56626 Andernach (DE)
(74) Representative: HOFFMANN - EITLE

(57) **Abstract**

This invention provides the use of a silicone-based transdermal therapeutic system having an area of 10 to 40 cm² and containing 0.1 to 3.15 mg / cm² of rotigotine as active ingredient, for the preparation of an anti-Parkinson medicament which induces a mean plasma concentration of rotigotine in the range of 0.4 to 2 ng/ml 24 h after administration.

## Description

### Field of the Invention

The present invention relates to an effective method for treating or alleviating symptoms of Parkinson's Disease, and the use of a Transdermal Therapeutic System (TTS) for delivering the dopamine receptor agonist rotigotine (INN) in a sufficient amount and at a sufficient rate to provide therapeutically effective treatment or alleviation of symptoms of Parkinson's disease.

### Technical Background

Parkinson's disease is believed to be primarily caused by the degeneration of dopaminergic neurons in the substantia nigra. This, in effect, results in loss of tonic dopamine secretion and dopamine-related modulation of neuronal activity in the caudate nucleus, and thus in a deficiency of dopamine in certain brain regions. The resulting imbalance of neurotransmitters acetylcholine and dopamine eventually results in disease related symptoms. Although usually regarded as a motor system disorder, Parkinson's Disease is now considered to be a more complex disorder that involves both motor and nonmotor systems. This debilitating disease is characterized by major clinical features including tremor, bradykinesia, rigidity, dyskinesia, gait disturbances, and speech disorders. In some patients, dementia may accompany these symptoms. Involvement of the autonomic nerve system may produce orthostatic hypotension, paroxysmal flushing, problems with thermal regulation, constipation, and loss of bladder and sphincter control.

Psychological disorders such as loss of motivation and depression may also accompany Parkinson's Disease.

Parkinson's Disease is primarily a disease of middle age and beyond, and it affects both men and women equally. The highest rate of occurrence of Parkinson's Disease is in the age group over 70 years old, where Parkinson's Disease exists in 1.5 to 2.5% of that population. The mean age at onset is between 58 and 62 years of age, and most patients develop Parkinson's Disease between the ages of 50 and 79. There are approximately 800,000 people in the United States alone with Parkinson's Disease.

Early motor deficits of Parkinson's Disease can be traced to incipient degeneration of nigral dopamine-releasing cells. This neuronal degeneration produces a defect in the dopamineric pathway that connects the substantia nigra to the striatum. As the disease progresses, refractory motor, autonomic, and mental abnormalities may develop, which implies that there is progressive degeneration of striatal receptor mechanisms.

The clinical diagnosis of Parkinson's Disease is based on the presence of characteristic physical signs. The disease is known to be gradual in onset, slowly progressive, and variable in clinical manifestation. Evidence suggests that the striatal dopamine content declines to 20% below levels found in age-matched controls before symptoms occur.

Treatment of Parkinson's disease has been attempted with, inter alia, L-dopa (levodopa), which still is the gold standard for the therapy of Parkinson's Disease. Levodopa passes the blood-brain barrier as a precursor for dopamine and is then converted into dopamine in the brain. L-dopa improves the symptoms of Parkinson's Disease but may cause severe side effects. Moreover, the drug tends to lose its effectiveness after the first two to three years of treatment. After five to six years, only 25% to 50% of patients maintain improvement.

Furthermore a major drawback of currently utilized therapies for Parkinson's Disease is the eventual manifestation of the "fluctuation syndrome", resulting in "all-or-none" conditions characterized by alternating "on" periods of mobility with dyskinesias and "off" periods with hypokinesia or akinesia. Patients who display unpredictable or erratic "on-off" phenomena with oral anti-Parkinson therapy have a predictable beneficial response to i.v. administration of L-dopa and other dopamine agonists, suggesting that fluctuations in plasma concentrations of drug are responsible for the "on-off" phenomena. The frequency of "on-off" fluctuations has also been improved by continuous infusions of the dopamine receptor agonists apomorphine and lisuride. However, this mode of administration is inconvenient. Therefore, other modes of administration providing a more constant plasma level, such as topical administration, are beneficial and have been suggested in the past.

As mentioned above, one treatment approach for Parkinson's disease involves dopamine receptor agonists. Dopamine receptor agonists (sometimes also referred to as dopamine agonists) are substances which, while structurally different from dopamine, bind to different subtypes of dopamine receptors and trigger an effect which is comparable to that of dopamine. Due to the reduced side-effects, it is advantageous when the substances selectively bind to a subgroup of dopamine receptors, i.e. the D2 receptors.

One dopamine receptor agonist which has been used to treat the symptoms of Parkinson's Disease is rotigotine. It has mostly been tested in the form of its hydrochloride. Rotigotine is the International Non-Proprietary Name (INN) of the compound (-)-5,6,7,8-tetrahydro-6-[propyl-[2-(2-thienyl)ethyl]-amino]-1-naphthalenol having the structure shown below

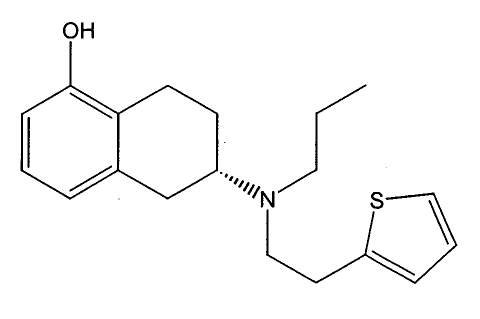

To date, various transdermal therapeutic systems (TTS) for the administration of rotigotine have been described. WO 94/07568 discloses a transdermal therapeutic system containing rotigotine hydrochloride as active substance in a two-phase matrix which is essentially formed by a hydrophobic polymer material as the continuous phase and a disperse hydrophilic phase contained therein and mainly containing the drug and hydrated silica. The silica enhances the maximum possible loading of the TTS with the hydrophilic salt. Moreover, the formulation of WO 94/07568 usually contains additional hydrophobic solvents, permeation-promoting substances, dispersing agents and, in particular, an emulsifier which is required to emulsify the aqueous solution of the active principle in the lipophilic polymer phase. A TTS, prepared by using such a system, has been tested in healthy subjects and Parkinson patients. The average drug plasma levels obtained by using this system were around 0.15 ng/ml with a 20 cm² patch containing 10 mg rotigotine. This level must be considered as too low to achieve a truly efficacious treatment or alleviation of symptoms related to Parkinson's disease.

Various further transdermal therapeutic systems have been described in WO 99/49852. The TTS used in this patent application comprise a backing layer, inert with respect to the constituents of the matrix, a self-adhesive matrix layer containing an effective quantity of rotigotine or rotigotine hydrochloride and a protective film which is to be removed before use. The matrix system is composed of a non-aqueous polymer adhesive system, based on acrylate or silicone, with a solubility of rotigotine of at.least 5% w/w. Said matrix is essentially free of inorganic silicate particles. In Examples 1 and 2 and in Figure 1 of WO 99/49852 two transdermal therapeutic systems are compared. These are based on acrylate or silicone adhesives, respectively. Figure 1 of WO 99/49852 shows that a silicone patch releases about the same amount of active principle through skin as an acrylate patch. This has been demonstrated by the almost identical drug flux rates in an in vitro model, independent of the adhesive test system employed. Therefore an identical flux rate through human skin was expected.

It should be noted that the drug content of the silicone patch used in WO 99/49852 was lower than the drug content used in the acrylate patch. However, this merely reflects the difference in solubility of the drug in the respective polymeric silicone and acrylate adhesives used in Examples 1 and 2, respectively. The TTS used in both examples contained the drug at about its saturation solubility in the respective adhesive systems. While the acrylate system is able to dissolve more drug than the silicone system, silicone in turn allows for a better release of the drug to skin. As these two effects compensate each other, it has been thought that the acrylate and the silicone system as used in WO 99/49852 are about equivalent in the obtainable drug plasma levels and, hence, in therapeutic efficacy.

Considering the rather discouraging experiences made with the silicone formulation of WO 94/07568, the acrylate-based TTS of Example 1 of WO 99/49852 has been subjected to clinical tests (safety and pharmacokinetic studies). The mean steady flux rate across human skin in vitro of this TTS amounted to 15.3 µg/cm²/h. However, it turned out that the plasma levels obtained using this TTS still is unsatisfactory and too low to allow for a really efficacious treatment of Parkinson's Disease. A 30 mg (20 cm²) patch only yielded a mean maximum plasma concentration of 0.12 ng/ml, while a 5 cm² patch containing 7.5 mg yielded a mean maximum plasma concentration of 0.068 ng/ml. Again, such values have to be considered as too low to provide a real therapeutic progress in the treatment of Parkinson's Disease. Thus, in summary, both the 20 cm² silicone patch of WO 94/07568 and the 20 cm² acrylate patch of WO 99/49852 failed to evoke sufficient drug plasma levels to provide a satisfactory therapeutic effectiveness.

In view of these experiences, it has been very surprising that a transdermal therapeutic system containing rotigotine in free base form in a silicone matrix could not only provide unexpectedly high plasma levels of rotigotine but also a significant therapeutic progress in the treatment of Parkinson's Disease. In particular, it has been observed that a silicone-based TTS containing rotigotine in the free base form provides mean maximum drug plasma levels in the range of almost 0.5 ng/ml for a 20 cm² silicone patch containing 9 mg of rotigotine. This is more than three times as much as could be expected from previous investigations.

Such plasma values are sufficient to allow for a reasonable expectation that an effective treatment of Parkinson's Disease with less side effects can be provided. It should be understood that the term "treatment" in the context of this application is meant to designate a treatment or alleviation of the symptoms of Parkinson's Disease, rather than a real causative treatment leading to a complete cure.

### Summary of the Invention

The present invention provides the use of a silicone-based transdermal therapeutic system having an area of 10 to 40 cm² and containing 0.1 to 3.15 mg / cm² of rotigotine as active ingredient, for the preparation of an anti-Parkinson medicament which induces a mean plasma concentration of rotigotine in the range of 0.4 to 2 ng/ml 24 h after administration.

The silicone-based transdermal therapeutic system as used in the present invention must contain at least one amine resistant silicone compound as the main component. Usually, the silicone compound will be a pressure sensitive adhesive or a mixture thereof and will form a matrix in which the other components of the TTS are embedded. Moreover, the adhesive(s) should preferably be pharmaceutically acceptable in a sense that it is biocompatible, non-sensitizing and non-irritating to skin. Particularly advantageous silicone adhesives for use in the present invention should further meet the following requirements:
- Retained adhesive and cohesive properties in the presence of moisture or perspiration, under normal temperature variations,
- good compatibility with rotigotine as well as with the further excipients used in the formulation; in particular, the adhesive should not react with the amino group contained in rotigotine.

It has been shown that pressure sensitive adhesives of the type forming a soluble polycondensed polydimethylsiloxane (PDMS) / resin network, wherein the hydroxy endgroups are capped with e.g. trimethylsilyl (TMS) groups, are particularly useful in the practice of the present invention. Preferred adhesives of this kind are the BIO-PSA silicone pressure sensitive adhesives manufactured by Dow Corning, particularly the Q7-4201 and Q7-4301 qualities. However, other silicone adhesives may likewise be used.

In further and preferred aspects the present invention also provides a silicone-based transdermal therapeutic system comprising two or more silicone adhesives as the main adhesive components for such use. It can be advantageous if such a mixture of silicone adhesives comprises at least one high tack and at least one medium tack adhesive to provide for the optimum balance between good adhesion and little cold flux. Excessive cold flux may result in a too soft patch which easily adheres to the package or to patient garments. Moreover, such a mixture of adhesives seems to be particularly useful for obtaining high plasma levels. A mixture of the aforementioned Q7-4201 (medium tack) and Q7-4301 (high tack) amine resistant silicone pressure sensitive adhesives in about equal amounts proved to be particularly useful in the practice of this invention.

In a further preferred embodiment, the silicone-based transdermal therapeutic system further includes a solubilizer. Several surfactant or amphiphilic substances may be used as solubilizers. They should be pharmaceutically acceptable and approved for use in medicaments. A particularly preferred example of such a solubilizer is soluble polyvinylpyrrolidone. Polyvinylpyrrolidone is commercially available, e.g. under the trademark Kollidon® (Bayer AG). Other examples include copolymers of polyvinylpyrrolidone and vinyl acetate, polyethyleneglycol, polypropyleneglycol, glycerol and fatty acid esters of glycerol or copolymers of ethylene and vinylacetate.

The silicone-based transdermal therapeutic system for use according to the present invention preferably contains less than 1 wt% of inorganic silicates, most preferably it is completely free from inorganic silicates.

The water content in the transdermal therapeutic systems for use in the present invention is preferably low enough so that no evaporation of water during preparation of the TTS is necessary. Typically, the water content in a freshly prepared patch is below 2%, more preferably 1 wt% or lower.

In a particularly preferred embodiment of the present invention, the transdermal therapeutic system has a surface area of 10 to 30 cm², more preferably 20 to 30 cm². It goes without saying that a TTS having a surface area of, say, 20 cm² is pharmacologically equivalent to and may be exchanged by two 10 cm² patches or four 5 cm² patches having the same drug content per cm². Thus, the surface areas as indicated in this application should be understood to refer to the total surface of all TTS simultaneously administered to a patient.

Providing and applying one or several transdermal therapeutic systems according to the invention has the pharmacological advantage over oral therapy that the attending physician can titrate the optimum dose for the individual patient relatively quickly and accurately, e.g. by simply increasing the number or size of patches given to the patient. Thus, the optimum individual dosage can often be determined after a time period of only about 3 weeks with low side effects.

A preferred content of rotigotine per patch is in the range of 0.1 to 2.0 mg / cm². Still more preferred are 0.4 to 1.5 mg rotigotine per cm². If a 7 day patch is desired, higher drug contents will generally be required. A rotigotine content in the range of about 0.4 to 0.5 wt% has been found to be particularly advantageous in that it provides the optimum usage of the drug contained in the TTS, i.e. there is only very little residual drug content in the TTS after administration. The apparent dose administered by using such a TTS usually is 50% or more and may be as high as 80-90% of the drug amount originally contained in the TTS.

The fact that the silicone-based transdermal therapeutic system described in this invention is able to provide a significant therapeutic effect against symptoms of Parkinson's Disease and high plasma levels of 0.4 ng/ml and more even at surface areas of 10 to 30 cm² and particularly as little as 10 or 20 cm² and at low drug contents of about 0.4 to 0.5 mg/cm², particularly about 0.45 g/cm², must be considered as a further particular benefit provided by the present invention.

The transdermal therapeutic system used in the present invention usually is a patch having a continuous adhesive matrix in at least its center portion containing the drug. However, transdermal equivalents to such patches are likewise comprised by the present invention, e.g. an embodiment where the drug is in an inert but non-adhesive silicone matrix in the center portion of the TTS and is surrounded by an adhesive portion along the patch edges.

In a further aspect, this invention relates to a method of treating Parkinson's Disease by applying on a patient in need thereof a silicone-based transdermal therapeutic system having an area of 10 to 40 cm² and containing 0.1 to 3.15 mg / cm² of rotigotine as active ingredient, wherein said transdermal therapeutic system induces a mean plasma concentration of rotigotine in the range of 0.4 to 2 ng/ml 24 h after administration.

Unless expressly indicated otherwise, any references to rotigotine in the context of this invention and the claims of this application mean rotigotine in the form of its free base. In some cases traces of rotigotine hydrochloride may be contained in a rotigotine preparation but these traces typically do not exceed 5 wt%, based on the amount of the free base. More preferably the content of hydrochloride impurities should be less than 2 wt%, even more preferably less than 1% and most preferably the rotigotine used in the present invention contains less than 0.1 wt% or no hydrochloride impurities at all.

As a result of the present invention it has been possible to achieve plasma levels which allow for a constant receptor stimulation of the dopamine receptors of Parkinson patients. In one embodiment of the invention, using one 20 cm² silicone patch prepared according to the preparation example below and containing 9 mg rotigotine resulted in a mean maximal plasma concentration of 0.491 ± 0.151 ng/ml at 23 h after start of administration. After 24 h, the mean plasma concentration was 0.473 ± 0.116 ng/ml. The individual maximal plasma concentration measured was 0.562 ± 0.191 ng/ml and calculated AUC(0-t) was 11.12 ± 4.05 ng/ml.

These parameters were determined in a pilot study involving 14 healthy male subjects who were administered either one or two of the silicone based transdermal therapeutic systems as described in the Preparatory Example, or an acrylic transdermal preparation according to WO 99/49852, respectively, in a single-center, open-label, single administration, three-way cross-over, partly randomized design. Individual drug plasma levels were determined by a validated routine LC-MS-MS assay, i.e. by a liquid chromatographic system equipped with a Tandem Mass Spectrometer having a limit of quantification of 10 pg/ml. The pharmacokinetic variables were the measured maximal concentration (Cₘₐₓ), the time of the observed maximum (tₘₐₓ) and AUC(0-t_{z}), i.e. the area under the concentration/time curve calculated by the linear trapezoidal rule up to the last sample with a quantifiable concentration. Based on the results in individual subjects, mean concentration, standard deviation, median and range were then determined and used for descriptive statistics of each parameter.

Moreover, the study revealed an approximately linear relationship between the drug amount administered to the subjects and the observed mean plasma concentrations of rotigotine. After administration of two silicone patches of the same kind as described above, the plasma concentrations increased by a factor of about 2 to 0.951 ± 0.309 ng/ml within 24 h.

This experimental study of which further details are given in the Examples below, confirms that it is realistic to expect mean plasma levels of rotigotine in the range of 0.4 to 2.0 ng/ml 24 h after administration of a silicone based transdermal therapeutic system having an area of 10 to 40 cm² and including 0.1 to 3.15 g/cm² rotigotine.

Additional clinical studies in male healthy volunteers have shown that the plasma levels obtainable according to the present invention are, by and large, maintained in vivo upon further (normally once daily) administration of the same transdermal therapeutic system for several weeks. For example, the mean plasma level obtained following a 3 months administration of a 20 cm² patch according to the present invention containing 9 mg rotigotine proved to be 0.49 ± 0.23 ng/ml. Thus the plasma levels as indicated herein with reference to a single administration and measured 24 hours thereafter, can be considered to represent steady state values. Thus, obtaining and maintaining high plasma levels of rotigotine for an extended period of time represents a further aspect of the present invention. The high steady-state concentration provided by the TTS according to the present invention is effective to avoid the on-off-fluctuations which typically accompany oral treatment.

The invention and the best mode for carrying it out will be explained in more detail in the following non-limiting examples.

### Preparation Example

A transdermal therapeutic system using a combination of silicone-type pressure sensitive adhesives was prepared as follows.

(-)-5,6,7,8-tetrahydro-6-[propyl-[2-(2-thienyl)ethyl]-amino]1-naphthalenol hydrochloride (rotigotine hydrochloride, 150 g) was added to a solution of 17.05 g NaOH in 218 g ethanol (96%). The resulting mixture was stirred for approximately 10 minutes. Then 23.7 g of sodium phosphate buffer solution (8.35 g Na₂HPO₄x2H₂O and 16.07 g NaH₂PO₄x2H₂O in 90.3 g water) was added. Insoluble or precipitated solids were separated from the mixture by filtration. The filter was rinsed with 60.4 g ethanol (96%) to obtain a particle-free ethanolic solution of Rotigotine in the form of the free base.

The Rotigotine free base solution (346.4 g) in ethanol (35% w/w) was mixed with 36.2 g ethanol (96%). The resulting solution was mixed with 109 g of an ethanolic solution containing 25 wt% polyvinylpyrrolidone (KOLLIDON® 90F), 0.077 wt% aqueous sodium bisulfite solution (10 wt%), 0.25 wt% ascorbyl palmitate, and 0.63 wt% DL-alpha-tocopherol until homogenous. To the mixture, 817.2 g of an amine resistant high tack silicone adhesive (BIO-PSA® Q7-4301 mfd. by Dow Corning) (74 wt% solution in heptane), 851.8 g of an amine resistant medium tack silicone adhesive (BIO-PSA® Q7-4201 mfd. by Dow Corning) (71 wt% solution in heptane), and 205.8 g petrol ether (heptane) were added, and all components were stirred until a homogenous dispersion was obtained.

The dispersion was coated onto a suitable polyester release liner (SCOTCHPAK® 1022) with a suitable doctor knife and the solvents were continuously removed in a drying oven at temperatures up to 80°C for about 30 min to obtain a drug-containing adhesive matrix of 50 g/m² coating weight. The dried matrix film was laminated with a polyester-type backing foil (SCOTCHPAK® 1109). The individual patches were punched out of the complete laminate in the desired sizes (e.g. 10 cm², 20 cm², 30 cm²) and sealed into pouches under the flow of nitrogen.

The following table shows the composition in mg/20 cm² of a transdermal therapeutic system according to the present invention containing a combination of two silicone-type PSA.

| Composition Components | Amount (mg) |
|---|---|
| Rotigotine Base | 9.00 |
| Polyvinylpyrrolidone | 2.00 |
| Silicone BIO-PSA® Q7-4301 | 44.47 |
| Silicone BIO-PSA® Q7-4201 | 44.46 |
| Ascorbyl palmitate | 0.02 |
| DL-alpha Tocopherol | 0.05 |
| Sodium metabisulfite | 0.0006 |
| Matrix coating weight | 50 g/m² |

### Clinical Trials

The above described transdermal therapeutic system was tested in a pharmacokinetic study for the comparative bioavailability and dose-proportionality after a single administration. The study involved 14 healthy male subjects who received one or two silicone-based or one acrylic-based transdermal preparations of rotigotine, respectively. 11 subjects completed the trial.

The study design involved the use of 20 cm² silicone patches each containing 9 mg rotigotine. This dosage was chosen based on the earlier experiences with the acrylate transdermal therapeutic system of WO 99/49852, because the same plasma levels as obtained using this acrylate patch were expected. The dosage level was approved by the Ethikkommission of the Ärztekammer Nordrhein. The subjects participating in the trial were advised before administration that no severe adverse effects which could be attributed to treatment with rotigotine had been observed in earlier clinical studies.

The silicone based transdermal therapeutic systems were compared with the acrylate TTS according to example 1 of WO 99/49852 including a 20 cm² patch containing 30 mg rotigotine. The study design was an open, partly randomised, three-way cross-over study involving a single administration according to the following schedule:

| Treatment I | Treatment II | Treatment III |
|---|---|---|
| Day 1 | Day 8 | Day 15 |
| non-randomized | randomized | randomized |
| one silicone TTS | two silicone TTS or one acrylate TTS | two silicone TTS or one acrylate TTS |

No placebo was given in this study. Whether treatment II and III involved administration of two silicone patches or one acrylate patch was determined at random.

Individual drug plasma levels were determined by a validated routine LC-MS-MS assay, i.e. by a liquid chromatographic system equipped with a Tandem Mass Spectrometer having a limit of quantification of 10 pg/ml. The pharmacokinetic variables were the measured maximal concentration (Cₘₐₓ), the time of the observed maximum (tₘₐₓ) and AUC(0-t_{z}), i.e. the area under the concentration/time curve calculated by the linear trapezoidal rule up to the last sample with a quantifiable concentration. Based on the results in individual subjects, mean concentration, standard deviation, median and range were then determined and used for descriptive statistics of each parameter.

### Results:

Using one silicone patch the mean plasma levels increased up to 0.473 ± 0.116 ng/ml within 24 h. The approximated lag time was 3 h. The maximum of mean measured plasma concentration after administration of one silicone patch was 0.491 ± 0.151 ng/ml 23 h after start of administration. The individual maximal plasma concentration was 0.562 ± 0.191 ng/ml and calculated AUC(0-t) was 11.12 ± 4.05 ng/ml. The terminal half-life after removal of one silicone patch was 5.3 ± 0.7 h.

After administration of two silicone patches the plasma concentrations increased to 0.951 ± 0.309 ng/ml within 24 h. The approximated lag time was 3 h. The maximum of mean measured plasma concentration after administration of two silicone patches was 1.076 ± 0.37 ng/ml 15 h after start of administration. The individual maximal plasma concentration was 1.187 ± 0.349 ng/ml and calculated AUC(0-t) was 23.73 ± 8.51 ng/ml h. The terminal half life of rotigotine after removal of two silicone patches was 5.1 ± 0.4 h.

Using one acrylic patch the plasma concentrations increased up to 0.197 ± 0.079 ng/ml within 24 h. The approximated lag-time was 4 h. The maximum of mean measured rotigotine plasma concentration after administration of one acrylic patch was 0.202 ± 0.095 ng/ml 23 h after start of the administration. The individual maximal plasma concentration was 0.228 ± 0.109 ng/ml and calculated AUC(0-t) was 4.15 ± 2.17 ng/ml·h. The terminal half life of rotigotine after removal of one acrylic patch was 4.9 ± 1.5 h.

The apparent dose measured after administration of one silicone patch (by determining the residual concentration in the patch after use) was 5.18 ± 1.23 mg. Corresponding doses after two silicone patches were 10.24 ± 2.74 mg, after one acrylic patch 2.56 ± 1.27 mg per 24 h. The parameters Cₘₐₓ or AUL(0-t) with the apparent dose show good correlation, i.e. there is an approximately linear relationship between the drug amount administered to the subjects and the observed mean plasma concentrations of rotigotine.

Due to the apparent equivalence, in the drug permeability across skin under in vitro conditions, of the 20 cm² silicone and acrylate patches tested in WO 99/49852, the much higher *in vivo* plasma concentrations obtained by using the silicone patches used in the present study must be considered as surprising.

The significantly higher plasma levels to which the present invention pertains are expected to have a pharmacological relevance. This became manifest in the study described above, even though only healthy volunteers, i.e. subjects with normal dopamine levels, participated who by definition could not benefit from this treatment. To the contrary, it became apparent that the treated healthy individuals experienced much more drug-related adverse events than was expected when the study was designed. In fact, each volunteer experienced at least one adverse event, most experienced several ones. All adverse events were of mild to severe intensity and were completely resolved at study end. However, in two of the 14 volunteers, adverse events were the reason for premature study termination. The most frequent adverse events observed were drowsiness, somnolescence, nausea, vomiting, and headache.

Had one known before that the silicone patch used in the study according to the present invention could or would result in such high plasma levels as were finally observed, the dose regimen in the above reported healthy volunteer study would have been selected much lower to avoid such adverse effects. On the other hand, Parkinson patients who suffer from a deficiency in dopamine levels, will easily tolerate and, in fact, benefit from such high plasma levels of a specific dopamine D2-receptor agonist such as rotigotine. Therefore, the increased plasma level of rotigotine obtained when using the silicone TTS, which forms a central aspect of the present invention, also bears a therapeutic significance. This result was subsequently confirmed in clinical trials involving Parkinson patients.

## Claims

1. The use of a silicone-based transdermal therapeutic system having an area of 10 to 40 cm² and containing 0.1 to 3.15 mg / cm² of rotigotine as active ingredient, for the preparation of an anti-Parkinson medicament which induces a mean plasma concentration of rotigotine in the range of 0.4 to 2 ng/ml 24 h after administration.

2. The use according to claim 1 wherein the silicone-based transdermal therapeutic system comprises two or more silicone adhesives as the main adhesive components.

3. The use according to claim 1 or 2 wherein the silicone-based transdermal therapeutic system further includes a solubilizer.

4. The use according to claim 3 wherein the solubilizer is polyvinylpyrrolidone.

5. The use according to any of the preceding claims wherein the silicone-based transdermal therapeutic system contains less than 1 wt% of inorganic silicates.

6. The use according to claim 5 wherein the silicone-based transdermal therapeutic system is free from inorganic silicates.

7. The use according to any of the preceding claims wherein the transdermal therapeutic system has an area of 10 to 30 cm².

8. The use according to any of the preceding claims wherein the transdermal therapeutic system contains 0.1 to 1.5 mg / cm² of rotigotine.

9. The use according to claim 1 wherein the transdermal therapeutic system is a patch having an area of 10 to 30 cm² and a content of rotigotine of 0.4 to 0.5 mg/cm² in an adhesive silicone-based matrix.

10. The use according to any of the preceding claims wherein the mean plasma concentration of rotigotine in the range of 0.4 to 2 ng/ml is maintained for at least 14 days upon continuous further administration of said transdermal therapeutic system.

11. A method of treating Parkinson's Disease by applying on a patient suffering from this disease a silicone-based transdermal therapeutic system having an area of 10 to 40 cm² and containing 0.1 to 3.15 mg / cm² of rotigotine as active ingredient, wherein said transdermal therapeutic system induces a mean plasma concentration of rotigotine in the range of 0.4 to 2 ng/ml 24 h after administration.
